(19) Europäisches Patentamt
European Patent Office
Office européen des brevets

(11) Veröffentlichungsnummer: **0 255 471 B1**

(12) **EUROPÄISCHE PATENTSCHRIFT**

(45) Veröffentlichungstag der Patentschrift: **09.09.92**

(51) Int. Cl.$^5$: **C07D 257/02**, A61K 49/00, C07F 5/00, C07F 13/00, C07F 9/547

(21) Anmeldenummer: **87730085.5**

(22) Anmeldetag: **24.07.87**

Die Akte enthält technische Angaben, die nach dem Eingang der Anmeldung eingereicht wurden und die nicht in dieser Patentschrift enthalten sind.

(54) **1,4,7,10-Tetraazacyclododecan-Derivate.**

(30) Priorität: **28.07.86 DE 3625417**

(43) Veröffentlichungstag der Anmeldung:
**03.02.88 Patentblatt 88/05**

(45) Bekanntmachung des Hinweises auf die Patenterteilung:
**09.09.92 Patentblatt 92/37**

(84) Benannte Vertragsstaaten:
**AT BE CH DE ES FR GB GR IT LI LU NL SE**

(56) Entgegenhaltungen:
**EP-A- 0 124 766**
**EP-A- 0 232 751**
**EP-A- 0 238 196**
**WO-A-86/02352**
**FR-A- 2 539 996**

**CHEMICAL ABSTRACTS, Band 97, Nr. 24, 13. Dezember 1982, Seite 617, Zusammenfassung Nr. 206960z, Columbus, Ohio, US; C.C. BRYDEN et al.: "Multinuclear NMR study of three aqueous lanthanide shift reagents: complexes with EDTA and two macrocyclic ligands", & RARE EARTHS MOD. SCI. TECHNOL. 1982, 3, 53-7**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Gries, Heinz, Dr.**
**Helmstedter Strasse 19**
**W-1000 Berlin 31(DE)**
Erfinder: **Radüchel, Bernd, Dr.**
**Gollanczstrasse 132**
**W-1000 Berlin 28(DE)**
Erfinder: **Speck, Ulrich, Dr.**
**Benedikter Strasse 50**
**W-1000 Berlin 28(DE)**
Erfinder: **Weinmann, Hans-Joachim, Dr.**
**Ahornstrasse 31**
**W-1000 Berlin 41(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die Erfindung betrifft den in den Patentansprüchen gekennzeichneten Gegenstand d.h. makrocyclische Komplexbildner, Komplexe und Komplexsalze, diese Verbindungen enthaltende Mittel, ihre Verwendung als Diagnostika und Therapeutika sowie Verfahren zur Herstellung dieser Verbindungen und Mittel.

Metallkomplexe sind schon zu Beginn der 50er Jahre als Kontrastmittel für die Radiologie in Betracht gezogen worden. Die damals eingesetzten Verbindungen waren aber derart toxisch, daß eine Verwendung beim Menschen nicht in Betracht kam. Es war daher durchaus überraschend, daß sich bestimmte Komplexsalze als ausreichend verträglich erwiesen haben, so daß eine routinemäßige Anwendung am Menschen für diagnostische Zwecke in Erwägung gezogen werden konnte. Als erster Vertreter dieser Substanzklasse hat sich das in der EP-A-71563 beschriebene Dimegluminsalz des Gd DTPA (Gadolinium-III-Komplex der Diethylentriaminpentaessigsäure) als Kontrastmittel für die Kernspintomographie bisher in der klinischen Prüfung an über 1.000 Patienten sehr gut bewährt. Der Schwerpunkt der Anwendung liegt bei Erkrankungen des Zentralnervensystems.

Ein wesentlicher Grund für die gute Verträglichkeit von Gd DTPA in der klinischen Anwendung liegt in der hohen Wirksamkeit bei der Kernspintomographie, insbesondere bei vielen Hirntumoren. Wegen seiner guten Wirksamkeit kann Gd DTPA mit 0,1 mmol/kg Körpergewicht sehr viel niedriger dosiert werden als beispielsweise Röntgenkontrastmittel in vielen Röntgenuntersuchungen.

Mit dem Gegenstand der Erfindung nächst vergleichbare makroacyclische Komplexbildner und insbesondere 1, 4, 7, 10-tetraza-cyclododecan-N', N'', N''', N'''' tetraessigsäure sind aus FR-A- 2539996, WO-A-8602352, C.A. 97 (1982), 206960Z und EP-A- 124766 bekannt.

Ähnliche Komplex bildner sind auch in den früheren Druckschriften EP-A- 232751 und EP-A- 238196 beschrieben.

Als weiterer Vertreter der Komplexsalze hat sich das in der DE-A-34 01 052 beschriebene Megluminsalz des Gd DOTA (Gadolinium-III-Komplex der 1,4,7,10-Tetraazacyclododecan-tetraessigsäure) für diagnostische Zwecke bewährt.

Nun besteht aber der Wunsch, Chelate auch höher dosiert einzusetzen. Das ist besonders zum Nachweis bestimmter Erkrankungen außerhalb des Zentralnervensystems mit Hilfe der Kernspintomographie (NMR-Diagnostik), ganz besonders aber bei der Verwendung von Chelaten als Röntgenkontrastmittel, der Fall.

Chelate können im Vergleich zu jodierten Röntgenkontrastmitteln eine Reihe von Vorteilen bieten:

a) Strahlenabsorption im höherenergetischen Bereich, damit Verminderung der Strahlenbelastung für den Patienten und Verbesserung der Voraussetzungen für die Energiesubtraktionsmethode.

b) Vermeidung der als "Kontrastmittelreaktionen" bekannten, unvorhersehbaren, zum Teil sogar lebensbedrohenden oder tödlichen sogenannten "allergieartigen" oder kardiovaskulären Nebenwirkungen der jodierten heutigen Röntgenkontrastmittel.

Voraussetzung dafür sind:

- hohe Konzentration strahlenabsorbierender Elemente in der Lösung (Röntgen) bzw. starke Beeinflussung der NMR-Signale
- eine für die Diagnostik geeignete Pharmakokinetik
- sehr feste Bindung der Metallionen in ausscheidbaren Komplexen auch unter in-vivo-Bedingungen
- gute Verträglichkeit der hochkonzentrierten, hochdosierten Komplexlösung
- geringes allergoides Potential aller Bestandteile des Kontrastmittels
- hohe Stabilität und Lagerfähigkeit der chemischen Bestandteile der Kontrast lösung.

Diese Anforderungen gelten in unterschiedlichem Maße und in unterschiedlicher weise, aber grundsätzlich für alle Anwendungen der genannten Komplexe in der in-vivo-Diagnostik sowie zum Teil in der Therapie.

Die erfindungsgemäßen Substanzen und die aus ihnen bereiteten Lösungen erfüllen die genannten Anforderungen in überraschender Weise. Sie besitzen eine starke und durch die Wahl geeigneter Metallatome an die jeweiligen Prinzipien der diagnostischen oder therapeutischen Methode (Röntgen, NMR, Ultraschall, Nuklearmedizin) anpassungsfähige Wirksamkeit.

Die erfindungsgemäßen Substanzen kommen zur Anwendung:

1. Für die NMR-Diagnostik in Form ihrer Komplexe mit den Ionen der Übergangsmetalle der Ordnungszahlen 21 bis 29, 42 und 44.

2. Für die NMR- und Röntgendiagnostik in Form ihrer Komplexe mit den Ionen der Lanthanidenelemente der Ordnungszahlen 57 bis 70.

3. Für die Ultraschall-Diagnostik eignen sich sowohl diejenigen Substanzen, die zur Anwendung in der NMR-Diagnostik als auch jene, die zur Anwendung in der Röntgen-Diagnostik bestimmt sind.

4. Für die Radiodiagnostik und Radiotherapie in Form ihrer Komplexe mit den Radioisotopen der Elemente der Ordnungszahlen 27, 29, 31, 32, 38, 39, 43, 49, 62, 64, 70 odere 77.

Auch ohne spezifische Maßnahmen erlaubt ihre Pharmakokinetik die Verbesserung der Diagnose zahlreicher Erkrankungen. Die Komplexe werden zum größten Teil unverändert und rasch wieder ausgeschieden, so daß insbesondere im Falle der Verwendung relativ toxischer Metallionen als Wirkprinzip trotz hoher Dosierung keine auf das Metall zurückzuführenden schädlichen Wirkungen beobachtet werden.

Die praktische Anwendung der neuen Komplexe und Komplexbildner wird auch durch deren ausreichende, oft sogar sehr gute chemische Stabilität erleichtert.

Ein weiterer wesentlicher Vorteil der beschriebenen Komplexe und Komplexbildner ist deren außerordentliche chemische Vielseitigkeit. Neben dem Zentralatom lassen sieh die Eigenschaften durch die Wahl vielfältiger Substituenten und/oder Salzbildner den Anforderungen an Wirksamkeit, Pharmakokinetik, Verträglichkeit, Handhabbarkeit usw. anpassen. So kann eine in der Diagnostik und Therapie sehr erwünschte Spezifität der Verbindungen für Strukturen im Organismus, für bestimmte biochemische Substanzen, für Stoffwechselvorgänge, für Zustände der Gewebe oder Körperflüssigkeiten, insbesondere durch die Kopplung an biologische Substanzen oder an Substanzen, die eine Interaktion mit biologischen Systemen aufweisen, erzielt werden. Solche für die Kopplung geeigneten Substanzen können niedermolekular (zum Beispiel Glucose, Aminosäuren, Fettsäuren, Gallensäuren, Porphyrine) oder hochmolekular (Polysaccharide, Proteine, Antikörper usw.) sein oder auch körperfremde Strukturen darstellen, die sich aber in spezifischer Weise im Körper verteilen oder mit Bestandteilen des Körpers reagieren. Die Nutzung derartiger Prinzipien wird umso eher möglich sein, je empfindlicher das Nachweisverfahren für ein Diagnostikum ist oder je wirksamer ein zum Beispiel radioaktiv markierter Komplex in der Therapie ist.

Die erfindungsgemäßen Substanzen können in Form ihrer Komplexe mit Radioisotopen, wie zum Beispiel $^{192}Ir$, auch in der Radiotherapie eingesetzt werden. Weiterhin sind die erfindungsgemäßen Komplexbildner selbst oder in Form schwacher Komplexe mit vorzugsweise körpereigenen Ionen ($Ca^{2+}$, $Mg^{2+}$, $Zn^{2+}$, $Fe^{2+/3+}$) zur Therapie von Schwermetallvergiftungen oder bestimmten Speicherkrankheiten geeignet.

Die erfindungsgemäßen makrocyclischen Verbindungen werden durch die allgemeine Formel I für 1,4,7,10-Tetraazacyclododecan-Derivate gekennzeichnet:

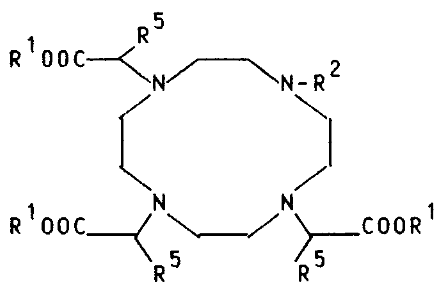 (I),

worin

- $R^1$ unabhängig voneinander Wasserstoff oder ein Metallionenäquivalent,
- $R^5$ Wasserstoff, eine Methyl- oder Ethylgruppe,
- $R^2$ eine gerade oder verzweigte, gesättigte oder ungesättigte, durch 1 bis 5 Hydroxy- oder $C_1$-$C_4$-Alkoxygruppen substituierte Alkylgruppe mit bis 16 Kohlenstoffatomen,
  $-CH_2-X-V$ mit X in der Bedeutung von Carbonyl, einer geradkettigen oder verzweigtkettigen Alkylengruppe mit 1 bis 10 Kohlenstoffatomen, die gegebenenfalls durch 1 bis 5 Hydroxy- oder $C_1$-$C_4$- Alkoxygruppen substituiert ist, oder einer gerad- oder verzweigtkettigen durch Sauerstoffatome unterbrochenen Alkylengruppe mit 2 bis 23 Kohlenstoffatomen,
  V in der Bedeutung von

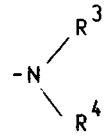 ,

wobei $R^3$ und $R^4$ unabhängig voneinander Wasserstoff, eine gerade oder verzweigte, gegebenenfalls durch 1 bis 5 Hydroxy- oder $C_1$-$C_4$-Alkoxygruppen substituierte Alkylgruppe mit bis 16 Kohlenstoffatomen oder $R^3$ und $R^4$ gemeinsam mit dem Stickstoffatom einen Pyrrolidin-, Piperidin-, Morpholin- oder Piperazinring darstellen,

oder

$R^2$ oder $R^3$ einen über eine 2-20 Kohlenstoffatome enthaltende Alkylenkette, die gegebenenfalls an den Enden Carbonylgruppen trägt und gegebenenfalls durch 1 bis 4 Sauerstoffatome unterbrochen oder durch 1 bis 5 Hydroxy-, $C_1$-$C_4$-Alkoxy- oder Carboxy-$C_1$-$C_4$-alkyl-Gruppen substituiert ist, gebundenen zweiten Makrozyklus der Formel I'

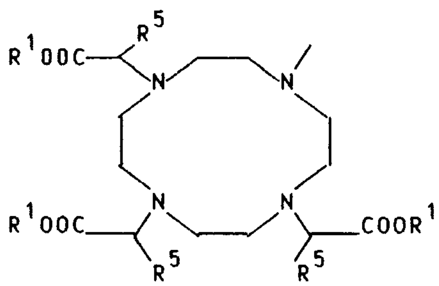

(I'),

oder

$R^2$ B oder $CH_2$-COB, wobei B für ein sich in dem zu untersuchenden Organ oder Organteil bzw. im Tumor besonders anreicherndes Makro- oder Biomolekül steht,

und im Molekül vorhandene funktionelle Gruppen gewünschtenfalls als Konjugat an Biomoleküle gekoppelt sind sowie deren Salze mit organischen oder anorganischen Basen oder Aminosäuren bzw. mit anorganischen oder organischen Säuren.

Verbindungen der allgemeinen Formel I mit $R^1$ in der Bedeutung von Wasserstoff werden als Komplexbildner und mit mindestens zwei der Substituenten $R^1$ in der Bedeutung eines Metallionenäquivalents werden als Metallkomplexe bezeichnet.

Als Alkylsubstituenten $R^2$, $R^3$, $R^4$ kommen gesättigte, ungesättigte, gerad- oder verzweigtkettige Kohlenwasserstoffe mit bis zu 16 C-Atomen, vorzugsweise gesättigte Kohlenwasserstoffe in Betracht, die im Falle von $R^3$ und $R^4$ gegebenenfalls, im Falle von $R^2$ definitionsgemäß, durch 1 bis 5 Hydroxy-oder niedere Alkoxygruppen substituiert sind.

Alkoxygruppen mit jeweils 1 bis 4 Kohlenstoffatomen sind insbesondere Methoxy- und Ethoxygruppen.

Als gegebenenfalls substituierte Alkylgruppen seien beispielsweise die Methyl-, Ethyl-, 2-Hydroxyethyl-, 2-Hydroxy-1-(hydroxymethyl)-ethyl-, 1-(Hydroxymethyl)ethyl-, Propyl-, Isopropenyl-, 2- und 3-Hydroxypropyl, 2,3-Dihydroxypropyl-, Butyl-, Isobutenyl-, 2-, 3-, und 4-Hydroxybutyl-, 2-, 3-und 4-Hydroxy2-methylbutyl-, 2- und 3-Hydroxy-isobutyl-, 2,3,4,-Trihydroxybutyl-und 2-Methoxyethylgruppe genannt.

Bevorzugt sind unsubstituierte Alkylgruppen mit 1 bis 7 Kohlenstoffatomen, wie zum Beispiel die Methyl-, Ethyl-, n-Propyl-, Isopropyl-, n-Butyl-, Isobutyl-, Pentyl- und Hexylgruppe. Ferner sind bevorzugt mono- und polyhydroxysubstituierte Alkylgruppen mit 2 bis 7 Kohlenstoffatomen und 1 bis 5, vorzugsweise 1 bis 4 Hydroxygruppen, wie zum Beispiel 2- und 3-Hydroxypropyl, 1,3-Dihydroxyisopropyl, 1-(Hydroxymethyl)-ethyl, Bis und Tris(hydroxymethyl)methyl, 2,3-Dihydroxy-1-hydroxymethylpropyl, 2,3,4,5,6-Pentahydroxyhexyl und vorzugsweise 2-Hydroxy-ethyl, 2-Hydroxy-1(hydroxymethyl)-ethyl, 2,3-Dihydroxypropyl und 2,3,4-Trihydroxybutyl.

Wenn $R^3$ und $R^4$ gemeinsam mit dem Stickstoffatom einen gegebenenfalls ein weiteres Heteroatom enthaltenden gesättigten Fünf- oder Sechsring darstellen, steht

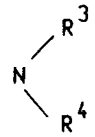

für Pyrrolidin, Piperidin, Morpholin oder Piperazin.

Die Alkylenkette, an die der zweite Makrocyclus I' gebunden ist, trägt an den Enden gegebenenfalls Carbonylgruppen und enthält 2 bis 20 C-Atome. Sie kann durch 1 bis 4 Sauerstoffatom(e) unterbrochen

oder durch 1 bis 5 Hydroxy-, $C_{1-4}$ Alkoxy-oder Carboxy-$C_{1-4}$-alkylgruppe(n) substituiert sein.

Alkoxy- und Alkylgruppen mit 1 bis 4 Kohlenstoffatomen sind insbesondere Methoxy-, Ethoxy-, Methyl- und Ethylgruppen.

Beispiele sind:

$-(CH_2)_2-$, $-CH_2-O-CH_2-$, $-(CH_2)_4-$, $-(CH_2-CH_2-O-CH_2-CH_2)-$, $-(CH_2O-CH_2)_2-$, $-(CH_2-O-CH_2)_3-$, $-CH_2-CH_2-(O-CH_2-CH_2)_3-$, $-CH_2-CH_2-(O-CH_2-CH_2)_4-$,

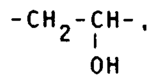

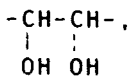 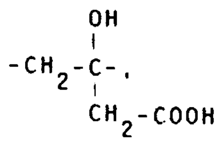 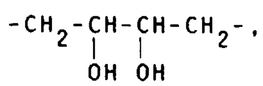

Wenn X eine Alkylengruppe mit 1-10 Kohlenstoffatomen darstellt, ist die Methylengruppe (1 C-Atom) bevorzugt.

Wenn nicht alle aciden Wasserstoffatome durch das Zentralion substituiert werden, können ein, mehrere oder alle verbleibenden Wasserstoffatom(e) durch Kationen anorganischer und/oder organischer Basen oder Aminosäuren ersetzt sein. Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins.

Die Komplexverbindungen können auch an Makromoleküle geknüpft sein, von denen bekannt ist, daß sie sich in dem zu untersuchenden Organ oder Organteil besonders anreichern. Solche Makromoleküle sind beispielsweise Hormone, Dextrane, Polysaccharide, Polychelone, Hydroxyethylstärke, Polyethylenglycol, Desferrioxamine, Bleomycine, Insulin, Prostaglandine, Steroidhormone, Aminozucker, Aminosäuren, Peptide, wie Polylysin, Proteine (wie z.B. Immunoglobuline und monoklonale Antikörper) oder Lipide (auch in Form von Liposomen). Besonders hervorzuheben sind Konjugate mit Albuminen, wie Humanserumalbumin, Antikörpern, wie z. B. monoklonale für tumorassoziierte Antigene spezifische Antikörper oder Antimyosin. Anstelle der Biomoleküle können auch geeignete synthetische Polymere wie Polyethylenimine angeknüpft werden. Die hieraus gebildeten diagnostischen Mittel eignen sich beispielsweise zur Anwendung in der Tumor- und Infarkt-Diagnostik. Als monoklonale Antikörper für die Konjugation kommen insbesondere solche in frage, die gegen überwiegend zellmembranständige Antigene gerichtet sind. Als solche sind zum Beispiel für die Tumordarstellung monoklonale Antikörper bzw. deren Fragmente ($F(ab)_2$) geeignet, die z.B. gegen das Carcinoembryonale Antigen (CEA), humanes Choriogonadotropin (ß-hCG) oder andere tumorständige Antigene, wie Glycoproteine, gerichtet sind. Geeignet sind u.a. auch Anti-Myosin, Anti-Insulin- und Anti-Fibrin-Antikörper.

Für Leberuntersuchungen bzw. für die Tumordiagnostik eignen sich beispielsweise Konjugate oder Einschlußverbindungen mit Liposomen (die beispielsweise als unilamellare oder multilamellare Phosphatidylcholin-Cholesterol-Vesikel eingesetzt werden).

Die Herstellung der makrocyclischen Verbindungen der allgemeinen Formel I erfolgt dadurch, daß man in an sich bekannter Weise in Verbindungen der allgemeinen Formel II

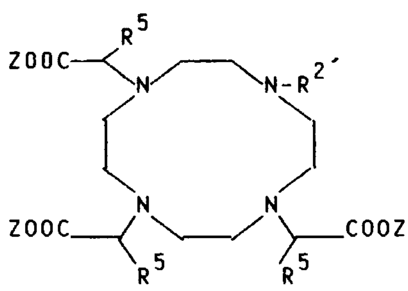 (II),

worin

$R^5$ die oben angegebene Bedeutung hat,

$R^{2'}$ die für $R^2$ angegebene Bedeutung hat, jedoch nicht für ein Makro- oder Biomolekül B oder die Gruppe $CH_2$-COB stehen soll und

Z Carboxylschutzgruppen bedeuten,

die Schutzgruppen Z abspaltet und die so erhaltenen Säuren ($R^1$ der allgemeinen Formel I steht für Wasserstoff) gewünschtenfalls

a) in an sich bekannter Weise mit mindestens einem Metalloxid oder Metallsalz eines Elements der Ordnungszahlen 21-29, 31, 32, 38, 39, 42-44, 49, 57-70 oder 77 umsetzt und anschließend, falls gewünscht, vorhandene acide Wasserstoffatome mit anorganischen und/oder organischen Basen oder Aminosäuren und vorhandene basische Gruppen mit anorganischen oder organischen Säuren in physiologisch verträgliche Salze überführt,

oder

b) in an sich bekannter Weise mit mindestens einem Metalloxid oder Metallsalz eines Elements der Ordnungszahlen 21-29, 31, 32, 38, 39, 42-44, 49, 57 bis 70 oder 77 umsetzt und anschließend die so erhaltenen Metallkomplexe in an sich bekannter Weise über im Molekül enthaltene funktionelle Gruppen oder an $R^2$ bzw. an die in $R^2$ enthaltene CO-Gruppe ein Makromolekül bindet und, falls gewünscht, vorhandene acide Wasserstoffatome mit anorganischen und/oder organischen Basen oder Aminosäuren und vorhandene basische Gruppen mit anorganischen oder organischen Säuren in physiologisch verträgliche Salze überführt,

oder

c) in an sich bekannter Weise über die im Molekül enthaltenen funktionellen Gruppen oder an $R^2$ bzw. an die in $R^2$ enthaltene CO-Gruppe ein Makromolekül bindet und anschließend in an sich bekannter Weise mit mindestens einem Metalloxid oder Metallsalz eines Elements der Ordnungszahlen 21-29, 31, 32, 38, 39, 42-44, 49, 57-70 oder 77 umsetzt und anschließend, falls gewünscht, vorhandene acide Wasserstoffatome mit anorganischen und/oder organischen Basen oder Aminosäuren und vorhandene basische Gruppen mit anorganischen oder organischen Säuren in physiologisch verträgliche Salze überführt.

Als Carboxylschutzgruppen Z kommen niedere Alkyl-, Aryl- und Aralkylgruppen infrage, beispielsweise die Methyl-, Ethyl-, Propyl-, Butyl-, Phenyl-, Benzyl-, Diphenylmethyl-, Triphenylmethyl-, bis(p-Nitrophenyl)-methylgruppe sowie Trialkylsilylgruppen.

Die Abspaltung der Schutzgruppen Z erfolgt in an sich bekannter Weise ,beispielsweise durch Hydrolyse, alkalische Verseifung der Ester, vorzugsweise mit Alkali in wäßrig-alkoholischer Lösung bei Temperaturen von 0 bis 50 °C oder im Falle von z. B. tert.-Butylestern mit Hilfe von Trifluoressigsäure.

Die Herstellung der Edukte erfolgt durch Cyclisierung zweier Reaktanten; die so erhaltenen cyclischen Verbindungen werden anschließend gegebenenfalls nach Abspaltung von Schutzgruppen, zur Einführung des Substituenten $R^2$ mit Halogenalkanen, -estern oder -säuren, umgesetzt.

Die Cyclisierung wird nach literaturbekannten Methoden, (zum Beispiel Org. Synth. 58, 86 (1978), Makrocyclic Polyether Syntheses, Springer Verlag Berlin, Heidelberg, New York 1982, Coord.Chem.Rev. 3,3 (1968), Ann. Chem. 1976,916) durchgeführt: einer der beiden Reaktanten trägt am Kettenende zwei Fluchtgruppen, der andere zwei Nukleophile, die diese Fluchtgruppen verdrängen. Als Beispiel seien genannt die Umsetzung von endständigen, ein Stickstoffatom enthaltenden, Dibrom-, Dimesyloxy-, Ditosyloxy- oder Dialkoxycarbonylalkylenverbindungen mit endständigen, ein zusätzliches Stickstoffatom in der Alkylenkette enthaltenden Diazaalkylenverbindungen.

Vorhandene Stickstoffatome sind gegebenenfalls geschützt, zum Beispiel als Tosylate, und werden vor der nachfolgenden Alkylierungsreaktion nach literaturbekannten Verfahren freigesetzt.
Werden Diester in die Cyclisierungsreaktion eingesetzt, so müssen die so erhaltenen Diketoverbindungen nach dem Fachmann bekannten Verfahren, zum Beispiel mit Diboran, reduziert werden.

Die nachfolgende Alkylierung erfolgt mit Halogenestern, -säuren oder -alkanen, die durch eine oder mehrere Hydroxy- oder niedere Alkoxygruppen substituiert sein können und gegebenenfalls Sauerstoffatom(e) in der Kette enthalten. Ferner kann der Alkylrest auch eine endständige Aminogruppe enthalten.

Wenn die Alkylierung mit einem dihalogenierten Alkan erfolgt, entstehen Verbindungen der allgemeinen Formel II mit zwei über eine Kohlenstoffbrücke verbundenen makrocyclischen Ringen.

Weitere literaturbekannte Verfahren zur Synthese von Verbindungen mit mehr als einem Ring sind zum Beispiel Reaktionen eines Amins mit einer Carbonylverbindung (zum Beispiel Säurechlorid, gemischtes Anhydrid, aktivierter Ester, Aldehyd); zweier aminsubstituierter Ringe mit einer Dicarbonylverbindung (zum Beispiel Oxalylchlorid, Glutardialdehyd); zweier Ringe, die je eine nukleophile Gruppe aufweisen, mit einer zwei Fluchtgruppen tragenden Alkylenverbindung; im Falle terminaler Acetyle oxidative Kupplung (Cadiot,

Chodkiewicz in Viehe "Acetylenes", 597-647, Marcel Dekker, New York, 1969).
Die die Ringe verknüpfende Kette kann anschließend durch Folgereaktionen modifiziert werden (zum Beispiel Hydrierung).

Die gegebenenfalls erforderliche Verseifung der bei der Alkylierung mit Halogenestern anfallenden Estergruppen wird nach den dem Fachmann bekannten Methoden durchgeführt (beispielsweise mit basischen Katalysatoren wie Alkali-oder Erdalkalicarbonaten oder -hydroxiden).

Bei der Alkylierung mit Halogenessigsäure erhält man ein Zwischenprodukt mit $R^2$ = $CH_2X$-COOH, das über das gemischte Anhydrid mit Chlorameisensäureester oder mit Hilfe von Dicyclohexylcarbodiimid und Reaktion mit einem primären oder sekundären Amin der allgemeinen Formel

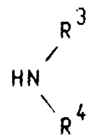

in das Monoamid überführt wird.

Als geeignete Amine seien beispielsweise genannt:
Dimethylamin, Diethylamin, Di-n-propylamin, Diisopropylamin, Di-n-butylamin, Diisobutylamin, Di-sek.butylamin, N-Methyln-propylamin, Dioctylamin, Pyrazolin, 2,3-Dihydroxypropylamin, N-Methyl-2,3-dihydroxypropylamin, 2-Hydroxyl-(hydroxymethyl)-ethylamin, N,N-Bis-(2-hydroxyethyl)-amin, N-Methyl-2,3,4,5,6-pentahydroxyhexylamin, 2-Hydroxyethylamin, 2-Amino-1,3-propandiol, Diethanolamin, Ethanolamin.

Die Polyhydroxyalkylamine können vorteilhafterweise auch in geschützter Form zur Reaktion eingesetzt werden, z.B. als O-Acylderivate oder als Ketale. Dies gilt besonders dann, wenn diese Derivate bequemer und billiger herstellbar sind als die Polyhydroxyalkylamine selbst. Ein typisches Beispiel ist das 2-Amino-1-(2,2-dimethyl-1,3-dioxolan-4-yl)-ethanol, das Acetonid des 1-Amino2,3,4-trihydroxybutans, hergestellt nach DE-A-31 50 917.

Die nachträgliche Entfernung der Schutzgruppen ist problemlos und kann z.B. durch Behandlung mit einem sauren Ionenaustauscher in wäßrig-ethanolischer Lösung erfolgen.

Die Verbindungen der allgemeinen Formel 1 mit $R^1$ in der Bedeutung eines Wasserstoffatoms stellen Komplexbildner dar. Sie können isoliert und gereinigt werden oder ohne Isolierung in Metallkomplexe der allgemeinen Formel I mit mindestens zwei der Substituenten $R^1$ in der Bedeutung eines Metallionenäquivalents überführt werden.

Die Herstellung der erfindungsgemäßen Metallkomplexe erfolgt in der Weise, wie sie in DE-A-34 01 052 und EP-A-71564 offenbart worden ist, indem man das Metalloxid oder ein Metallsalz (beispielsweise das Nitrat, Acetat, Carbonat, Chlorid oder Sulfat) des Elements der Ordnungszahlen 21-29, 31, 32, 38, 39, 42-44, 49, 57-70 oder 77 in Wasser und/oder einem niederen Alkohol (wie Methanol, Ethanol oder Isopropanol) löst oder suspendiert und mit der Lösung oder Suspension der äquivalenten Menge der komplexbildenden Säure der allgemeinen Formel I mit $R^1$ in der Bedeutung eines Wasserstoffatoms umsetzt und anschließend, falls gewünscht, vorhandene acide Wasserstoffatome von Säuregruppen durch Kationen anorganischer und/oder organischer Basen oder Aminosäuren substituiert.
Die Neutralisation erfolgt dabei mit Hilfe anorganischer Basen (zum Beispiel Hydroxiden, Carbonaten oder Bicarbonaten) von zum Beispiel Natrium, Kalium oder Lithium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin.

Zur Herstellung der neutralen Komplexverbindungen kann man beispielsweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol. Ethanol, Isopropanol und anderen), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man die komplexbildende Säure in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Basische Gruppen können mit anorganischen und/oder organischen Säuren in pharmazeutisch verträgliche Salze überführt werden.

Als anorganische Basen werden beispielsweise Lithium-, Natrium-und Kaliumhydroxid verwendet. Als organische Basen sind unter anderem primäre, sekundäre und tertiäre Amine, wie zum Beispiel Ethanolamin, Morpholin, Glucamin, N-Methyl- und N,N-Dimethylglucamin, sowie basische Aminosäuren, wie zum Beispiel Lysin, Arginin und Ornithin, geeignet.

Zur Salzbildung mit basischen Gruppen kommen anorganische Säuren, wie zum Beispiel Salzsäure, und organische Säuren, wie zum Beispiel Zitronensäure, infrage.

Die Konjugatbildung kann z.B. über eine Carboxylgruppe der Komplexverbindung oder über eine funktionelle Gruppe erfolgen. Es können bei der Konjugatbildung der Säuren mit Makromolekülen mehrere Säurereste an dieses gebunden werden. In diesem Fall können mehrere Zentralionen an ein Makromolekül gebunden werden.

Die Kopplung an die gewünschten Makromoleküle erfolgt ebenfalls nach an sich bekannten Methoden, wie sie z. B. in Rev. roum. Morphol. Embryol. Physiol., Physiologie 1981, 18, 241 und J. Pharm. Sci. 68, 79 (1979) beschrieben sind, beispielsweise durch Reaktion der nucleophilen Gruppe eines Makromoleküls, wie der Amine-, Phenol-, Sulfhydryl-, Aldehyd- oder Imidazol-Gruppe mit einem aktivierten Derivat des Komplexbildners. Als aktivierte Derivate kommen beispielsweise Monoanhydride, Säurechloride, Säurehydrazide, gemischte Anhydride (s. z.B. G.E. Krejcarek und K.L. Tucker, Biochem. Biophys. Res. Commun. 1977, 581), aktivierte Ester, Nitrene oder Isothiocyanate in Betracht.
Umgekehrt ist es auch möglich, ein aktiviertes Makromolekül mit der komplexbildenden Säure umzusetzen. Zur Konjugation mit Proteinen bieten sich auch Substituenten zum Beispiel der Struktur $C_6H_4N_2$, $C_6H_4NHCOCH_2$, $C_6H_4NHCS$ oder $C_6H_4OCH_2CO$ an.

Die Konjugation der komplexbildenden Säure mit Dextranen und Dextrinen erfolgt ebenfalls nach an sich bekannten Methoden, beispielsweise durch Aktivierung der Polysaccharide mit Bromcyan und anschließende Umsetzung mit Aminogruppen der komplexbildenden Säure.

Im Falle der Verwendung von Radioisotope enthaltenden Komplexverbindungen kann deren Herstellung nach den in "Radiotracers for Medical Applications", Volume 1, CRC-Press, Boca Raton, Florida beschriebenen Methoden vorgenommen werden.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentaessigsäure) oder, falls erforderlich, Elektrolyte wie zum Beispiel Natriumchlorid oder, falls erforderlich, Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween(R), Myrj(R) und/oder Aromastoff(en) zur Geschmackskorrektur (zum Beispiel ätherischen Olen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kentrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 1μMol - 1 Mol/l des Komplexsalzes und werden in der Regel in Mengen von 0,001 - 5 mMol/kg dosiert. Sie sind zur enteralen und parenteralen Applikation bestimmt.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler

Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der erfindungsgemäßen Mittel erlaubt es hochkonzentrierte Lösungen herzustellen, damit die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität in-vitro auf, sondern auch eine überraschend hohe Stabilität in- vivo, so daß eine Freigabe oder ein Austausch der in den Komplexen nicht kovalent gebundenen - an sich giftigen -Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,001 - 5 mMol/kg, vorzugsweise 0,005 - 0,5 mMol/kg, dosiert. Details der Anwendung werden zum Beispiel in H.J. Weinmann et al., Am. J. of Roentgenology 142, 619 (1984) diskutiert.

Besonders niedrige Dosierungen (unter 1 mg/kg) von organspezifischen NMR-Diagnostika sind z.B. zum Nachweis von Tumoren und von Herzinfarkten einsetzbar. Ferner können die erfindungsgemäßen Komplexverbindungen vorteilhaft als Shift-Reagenzien verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida beschrieben.

Eine weitere bildgebende Methode mit Radioisotopen ist die Positronen-Emissions-Tomographie, die positronenemittierende Isotope wie z.B. $^{43}Sc$, $^{44}Sc$, $^{52}Fe$, $^{55}Co$ und $^{68}Ga$ verwendet. (Heiss, W.D., Phelps, M.E., Position Emission Tomography of Brain, Springer Verlag Berlin, Heidelberg, New York 1983).

Die erfindungsgemäßen Verbindungen können auch in der Radioimmunotherapie verwendet werden. Diese unterscheidet sich von der entsprechenden Diagnostik nur durch die Menge und Art des verwendeten radioaktiven Isotops. Ziel ist dabei die Zerstörung von Tumerzellen durch energiereiche kurzwellige Strahlung mit einer möglichst geringen Reichweite. Die Spezifität des verwendeten Antikörpers ist dabei von entscheidender Bedeutung, da unspezifisch lokalisierte Antikörperkonjugate zur Zerstörung von gesundem Gewebe führt.

Der Antikörper bzw. das Antikörper-Fragment des erfindungsgemäßen Antikörper-Metall-Komplexes dient dazu, den Komplex immunspezifisch für das betreffende Antigen an das Zielorgan zu transportieren, wo das wegen seiner zelltötenden Eigenschaften ausgewählte Metallion Strahlen emittieren kann, die die Zellen lethal schädigen. Geeignete $\beta$-emittierende Ionen sind, zum Beispiel $^{46}Sc$, $^{47}Sc$, $^{48}Sc$, $^{72}Ga$ und $^{73}Ga$. Geeignete geringe Halbwertszeiten aufweisende $\alpha$-emittierende Ionen sind zum Beispiel $^{211}Bi$, $^{212}Bi$, $^{213}Bi$ und $^{214}Bi$, wobei $^{212}Bi$ bevorzugt ist.

Bei der in- vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden parenteral, vorzugsweise i.V. appliziert.
Details der Anwendung von Radiotherapeutika werden zum Beispiel in R.W. Kozak et. al. TIBTEC, October 1986, 262 diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktienstechniken.

Im allgemeinen werden die erfindungsgemäßen Mittel für die Anwendung als Röntgenkontrastmittel in Analogie zu zum Beispiel Meglumin-Diatrizoat in Mengen von 0,1 -5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler - "Einführung in die Röntgendiagnostik", G. Thieme, Stuttgart, New York (1977) diskutiert.

Die erfindungsgemäßen Mittel sind - da ihre akustische Impedanz höher ist als die von Körperflüssigkeiten und Geweben - auch als Kontrastmittel für die Ultraschall-Diagnostik geeignet, insbesondere in Form von Suspensionen. Sie werden im allgemeinen in Mengen von 0,1 bis 5 mMol/kg, vorzugsweise von 0,25

bis 1 mMol/kg, dosiert.

Details der Anwendung von Ultraschalldiagnostika werden z.B. in T.B. Tyler et al., Ultrasonic Imaging 3.323 (1981), J.I. Haft, "Clinical Echocardiography", Futura, Mount Kisco, New York 1978 und G. Stefan "Echokardiographie" G. Thieme Stuttgart/New York 1981, beschrieben.

Insgesamt ist es gelungen, neue Komplexbildner, Metallkomplexe und Metallkomplexsalze zu synthetisieren, die neue Möglichkeiten in der diagnostischen und therapeutischen Medizin erschließen. Vor allem die Entwicklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstandes.

Im folgenden wird beispielsmäßig die Synthese von Ausgangsmaterial, ausgehend von einer Cyclisierungsreaktion, beschrieben:

a) 1-Benzyl-4,7,10-tris-(p-tolylsulfonyl)-1,4,7,10-tetraazacyclododecan

Zu einer Lösung von 164,6 g N,N',N''-Tris-(p-tolylsulfonyl)diethylentriamin-N,N''-dinatriumsalz in 2,16 l Dimethylformamid tropft man bei 100 °C unter Rühren 145 g N,N-Bis- [2,2'-(p-tolylsulfonyloxy)]-ethanbenzylamin, gelöst in 900 ml Dimethylformamid innerhalb von 3 Stunden zu. Man tropft dann unter Rühren bei 80 °C 1 l Wasser hinzu und rührt 18 Stunden weiter bei Raumtemperatur, kühlt dann auf 0 °C und saugt den Niederschlag ab, wäscht mit wenig eiskaltem Ethanol und trocknet bei 15 Torr und 60 °C. Man erhält 175 g der Titelverbindung.

Alternative Route 1

Eine analoge Methode zur Herstellung von Tetraazacyclododecanderivaten findet sich bei M. Hediger und T.A. Kaden, Helv. Chim. Acta 66,861(1983).

30,94 g N,N',N''-Tris-(p-Tolylsulfonyl)-diethylentriamin-N,N''-dinatriumsalz und 28,12 g N-Bis-(2-Methansulfonyloxy-ethyl)-triphenylmethylamin werden mit 530 ml Dimethylformamid 20 Stunden bei 80-85°C gerührt, dann abgekühlt und in eine Lösung von 30 g Kaliumcarbonat in 5 l Eiswasser eingerührt. Man saugt den Niederschlag ab, wäscht mit 0,5 l Wasser den Filterkuchen und trocknet bei 20°C im Vakuum bei 150 Torr. Zur Reinigung löst man in 230 ml Chloroform und 5 ml Triethylamin, filtriert, engt im Vakuum auf 200 ml ein und versetzt die Lösung in der Siedehitze mit 250 ml Ethylacetat. Man läßt über Nacht abkühlen und saugt die ausgefallenen Kristalle ab. Man erhält 22, 18 g 1,4,7Tris-(p-Tolylsulfonyl)-10-triphenylmethyl-1,4,7,10-tetraazacyclododecan,
Fp.: 185-188°C (Zersetzung)

Zur Abspaltung der Tritylschutzgruppe rührt man 31,4 g des auf diese Weise hergestellten Tritosyl-tritylderivates in einer Mischung aus 100 ml Eisessig, 75 ml Wasser und 300 ml Dioxan 1 Stunde bei 80°C, engt dann im Vakuum bei 60°C weitgehend ein, verdünnt mit 300 ml Eiswasser und versetzt mit 40 ml 11 N Natronlauge (pH oberhalb 12). Diese Mischung wird mit 300 ml Chloroform geschüttelt, die Phasen getrennt, die Wasserphase zweimal mit je 100 ml Chloroform extrahiert und die vereinigten Chloroformphasen über Natriumsulfat getrocknet und im Vakuum eingedampft. Den schaumigen Rückstand behandelt man mit 300 ml Diethylether, wobei Kristallisation eintritt. Man saugt ab, trocknet die Kristalle im Vakuum bei 60°C und 150 Torr und erhält 21 g 1,4,7-Tris-(p-Tolylsulfonyl)-1,4,7,10-tetraazacyclododecan,
Fp.: 202-203°C.

Die so erhaltene Tritosylverbindung (21 g) löst man in 200 ml Dimethylformamid, versetzt die Lösung nacheinander mit 13,71 g wasserfreiem Kaliumcarbonat, 4,95 g Natriumjodid und 7,92 g Benzylbromid und rührt 5 Stunden bei 100°C. Man kühlt dann auf 20°C ab, rührt die Mischung in 4 l Eiswasser ein, saugt ab und löst den Rückstand in 2 l Dichlormethan. Die Lösung wird mit 100 ml Wasser extrahiert, über Natriumsulfat getrocknet und im Vakuum eingedampft. Den Rückstand löst man in der Siedehitze in 500 ml Acetonitril, läßt über Nacht auskristallisieren, saugt ab und trocknet die Kristalle bei 50°C und 150 Torr. Man erhält 16,20 g 1-Benzyl-4,7,10-tris-(p-tolylsulfonyl)-1,4,7,10-tetraazacyclodedecan,
Fp.: 217-219°C

b) N-Benzyl-1,4,7,10-tetraazacyclododecan

150 g 1-Benzyl-4,7,10-tris-(p-tolylsulfonyl)-1,4,7,10-tetraazacyclododecan werden mit 900 ml HBr/Essigsäure (40 %ig) und 125 g Phenol 16 Stunden auf 50 °C erhitzt. Nach Abkühlen auf 20 °C verdünnt man mit 1 l Ether, kühlt auf -5 °C und saugt die ausgefallenen Kristalle ab. Zur Isolierung der freien Base löst man das Produkt in 500 ml 4 N Natronlauge, sättigt mit Kaliumcarbonat und extrahiert

mehrmals mit Chloroform, trocknet über Magnesiumsulfat und dampft im Vakuum ein. Man erhält 39 g der Titelverbindung als hellgelbes zähes Öl. Eine Probe wurde als Tri-hydrochlorid charakterisiert: Schmelzpunkt 210 ° C (unter Zersetzung).

Alternative Route 2

Man versetzt eine Lösung von 11,2 g 1,4,7,10-Tetraazacyclododecan in 900 ml Tetrahydrofuran bei -20 ° C mit 58 ml Triethylamin und tropft unter Rühren innerhalb von 3 Stunden eine Lösung von 16,2 ml Benzoylchlorid in 280 ml Tetrahydrofuran zu, wobei die Zimmertemperatur leicht über -10 ° C steigt. Man rührt dann 16 Stunden bei 0 - 10 ° C, filtriert vom Niederschlag ab und dampft die Lösung im Vakuum ein. Den Rückstand chromatographiert man an 1 kg Kieselgel und eluiert mit Dioxan-Wasser-Ammoniak-Lösung (8:1:1). Die nach DSC einheitlichen Fraktionen werden vereinigt, eingedampft, in Dichlormethan gelöst und zur Entfernung einer geringen Trübung filtriert und die Lösung eingedampft. Man erhält 19,60 g 1,4,7-Tribenzoyl-1,4,7,10-tetraazacyclododecan, Fp.: 120-125 ° C.

11,5 g des so erhaltenen Tribenzoats werden in 150 ml Dimethylformamid mit 8,3 g wasserfreiem Kaliumcarbonat, 3,0 g Natriumjodid und 7,2 ml Benzylbromid 15 Stunden bei 100 ° C gerührt. Man filtriert, dampft die Lösung im Vakuum ein, rührt den Rückstand zweimal mit je 50 ml Hexan und dekantiert. Die Hexanphasen werden verworfen. Zur Reinigung löst man in Dichlormethan und chromatographiert mit Dichlormethan-Methanol (37:3) über 0,5 kg Kieselgel. Man erhält 10,2 g 1,4,7-Tribenzoyl-10-benzyl-1,4,7,10-tetraazacyclododecan, Fp.: 105-109 ° C.

Zur Abspaltung der Benzoylgruppen löst man dieses Produkt (2,87 g) in 290 ml Tetrahydrofuran, versetzt mit 11,2 g Kalium-t-butylat und erhitzt 48 Stunden unter Rückfluß. Man filtriert, dampft im Vakuum ein, versetzt den Rückstand unter Eiskühlung mit 100 ml Wasser und extrahiert dreimal mit je 50 ml Dichlormethan. Die vereinigten Dichlormethanphasen werden mit 10 ml Wasser geschüttelt, über Natriumsulfat getrocknet und im Vakuum eingedampft. Der zunächst ölige Rückstand kristallisiert sehr langsam und wird mit 20 ml Hexan verrieben. Nach Absaugen und Trocknen erhält man 1,15 g N-Benzyl-1,4,7,10-tetraazacyclododecan, Fp.: 75-78 ° C.

c) 1-Benzyl-4,7,10-tris-(ethoxycarbonylmethyl)-1,4,7,10-tetraazacyclododecan

Zu einer Lösung von 131,8 g N-Benzyl-1,4,7,10-tetraazacyclodedecan in 1,5 l Dichlormethan gibt man bei 0 ° C nacheinander 200 g Triethylamin und innerhalb von 2 Stunden 280 g Bromessigsäureethylester. Man rührt noch 16 Stunden bei Raumtemperatur, schüttelt mit 5 %iger Natriumcarbonatlösung und Sole, trocknet über Magnesiumsulfat und dampft im Vakuum ein. Den Rückstand löst man in 200 ml Chloroform und filtriert über 2 kg Kieselgel, wobei man mit 1 1 Chloroform-Methanol (95:5) eluiert. Man erhält 210 g der Titelverbindung als zähes Ol.

d) N,N'N''-Tris-(ethoxycarbonylmethyl)-1,4,7,10-tetraazacyclododecan

100 g 1-Benzyl-4,7,10-tris-(ethoxycarbonylmethyl)-1,4,7,10-tetraazacycledodecan werden in 0,5 l Essigsäure und 0,5 l Ethylacetat gelöst, mit 5 g Palladium auf Kohle (10 %) versetzt und 5 Stunden unter Wasserstoff geschüttelt. Man filtriert vom Katalysator ab, engt im Vakuum ein, löst den Rückstand in 1l Chloroform, schüttelt mit 100 ml gesättigter Sodalösung und 100 ml Sole, trocknet und dampft im Vakuum ein. Den Rückstand reinigt man durch Kugelrohrdestillation bei $10^{-3}$ Torr und 120 ° C. Man erhält N,N',N''-Tris(ethoxycarbonylmethyl)-1,4,7,10-tetraazacyclododecanals ein zähes hellgelbes Ol.
Ausbeute: 65 g.
IR (Film): 3400, 2935, 2878, 1738 /cm.

Beispiel 1

Gadolinium-III-Komplex der N-[2,3-Dihydroxy-N-methyl-propyl-carbamoylmethyl) 1,4,7,10-tetraazacyclododecan-N',N'',N'''-triessigsäure

55 g N-[2,3-Dihydroxy-N-methyl-propylcarbamoylmethyl]-N',N'',N'''-tris-(ethoxycarbonylmethyl)-1,4,7,10-tetraazacyclododecan löst man in 0,5 l Ethanol, versetzt mit 96 ml 3 N Natronlauge und rührt 3 Stunden bei 20 ° C, engt dann im Vakuum ein, versetzt mit 300 ml Wasser und stellt mit 2 N Salzsäure pH 6 ein. Zu dieser Lösung gibt man 31,94 g Gadoliniumacetat und rührt 18 Stunden bei 50 ° C, gibt dann über einen Anionenaustauscher Amberlite IRA 410 und anschließend das wäßrige Eluat über einen Kationenaustauscher Amberlite IRC 50.

Das Eluat wird im Vakuum eingedampft und getrocknet. Man erhält 47,14 g (73 % der Theorie) der Titelverbindung als farbloses Pulver.

| Analyse: $C_{20}H_{34}$ Gd $N_5O_9$ (645.77) | | | |
|---|---|---|---|
| C 37.20<br>37.52 | H 5.31<br>5.19 | Gd 24.35<br>24.09 | N 10.85 (ber.)<br>10.87 (gef.) |

Das Ausgangsmaterial für die Herstellung der Titelverbindung gemäß Beispiel 2 erhält man folgendermaßen:

a) N,N',N''-Tris-(ethoxycarbonylmethyl)-1,4,7,10-tetraazacyclododecan-N'''-essigsäure

Man löst 20 g N,N',N''-Tris-(ethoxycarbonylmethyl)-1,4,7,10-tetraazacyclododecan in 300 ml Dichlormethan, versetzt mit 10,40 g Triethylamin und tropft anschließend bei 0 °C eine Lösung von 4,40 g Chloressigsäure in 100 ml Dichlormethan hinzu und rührt 20 Stunden bei Raumtemperatur. Zur Aufarbeitung verteilt man zwischen Dichlormethan und Phosphatpuffer pH 6, trocknet über Magnesiumsulfat und dampft im Vakuum ein. Man erhält 23 g der gewünschten Verbindung als zähes Öl.

b) N-[2,3-Dihydroxy-N-methyl-propylcarbamoylmethyl]-N',N'',N'''tris-(ethoxycarbonylmethyl)-1,4,7,10-tetraazacyclododecan

Zu einer Lösung von 48,86 g (100 mMol) N,N',N''-Tris-(ethoxycarbonylmethyl)-1,4,7,10-tetraazacyclododecan-N'''essigsäure in 500 ml Dichlormethan tropft man bei 0 °C 10,5 g Triethylamin und anschließend eine Lösung von 14 g Chlorameisensäureisobutylester. Man rührt 1 Stunde bei 0 °C und tropft dann eine Lösung von 10,52 g N-Methylamino-2,3propandiol in 100 ml Chloroform zu und rührt 2 Stunden bei Raumtemperatur. Anschließend schüttelt man mit Natriumhydrogencarbonatlösung und Sole, trocknet über Magnesiumsulfat und dampft im Vakuum ein. Zur Reinigung filtriert man die Chloroformlösung des Rückstands über 500 g Kieselgel und erhält 55 g der Titelverbindung.

Beispiel 2

Gadolinium-III-Komplex der N-[N-Ethylcarbamoylmethyl]-1,4,7,10 tetraazacyclododecan-N',N'',N'''-triessigsäure

24 g N-[N-Ethylcarbamoylmethyl]-N',N'',N'''-tris-(ethoxycarbonylmethyl)-1,4,7,10-tetraazacyclodedecan löst man in 250 ml Ethanol, versetzt mit 47 ml 3 N Natronlauge und rührt 3 Stunden bei 20 °C, engt im Vakuum ein, versetzt mit 150 ml Wasser und stellt mit 2 N Salzsäure auf pH 6 ein. Man versetzt mit 15,55 g Gadoliniumacetat und rührt 5 Stunden bei 60°C. Die Lösung wird dann wie in den vorhergehenden Beispielen beschrieben über Ionenaustauscher gereinigt. Man erhält 20,39 g der Titelverbindung als farbloses Pulver.

| Analyse: $C_{18}H_{30}$ Gd $N_5O_7$ (585.72) | | | |
|---|---|---|---|
| C 36.91<br>36.98 | H 5.16<br>5.28 | Gd 26.85<br>26.58 | N 5.16 (ber.)<br>5.07 (gef.) |

Herstellung des Ausgangsmaterials:

Man löst 25 g (51,2 mMol) N,N',N''-Tris-(ethoxycarbonylmethyl)1,4,7,10-tetraazacyclododecan-N'''-essigsäure (siehe Beispiel 2a) in 200 ml Dichlormethan, versetzt bei 0 °C mit 5,40 g Triethylamin und anschließend mit 7 g Chlorameisensäureisobutylester. Man rührt 1 Stunde bei 0 °C und tropft dann eine Lösung von 2,31 g Ethylamin in 20 ml Dichlormethan hinzu, rührt 2 Stunden bei Raumtemperatur, schüttelt mit gesättigter Natriumhydrogencarbonatlösung und Sole, trocknet über Magnesiumsulfat und dampft im Vakuum zur Trockne. Zur Reinigung filtriert man die Dichlormethanlösung des Rückstands über 200 g

Kieselgel und erhält 24 g N-[N-Ethylcarbamoylmethyl]-N',N'',N'''-tris-(ethoxycarbenylmethyl)-1,4,7,10-tetraazacycledodecan.

Beispiel 3

Gadolinium-III-Komplex der N-(2,3-Dihydroxy-1-propyl)-1,4,7,10 tetraazacyclododecan-N',N'',N'''-triessigsäure

Man löst 4,30 g N,N',N''-Tris-(ethoxycarbonylmethyl)-1,4,7,10 tetraazacyclododecan in 100 ml Dichlormethan, versetzt mit 4,20 g Triethylamin und 2,21 g 3-Chlor-1,2-propandiol. Man rührt 16 Stunden bei Raumtemperatur, schüttelt mit Wasser und dampft im Vakuum ein. Den Rückstand rührt man 6 Stunden mit 100 ml 1N Natronlauge, stellt mit 2 N Salzsäure auf pH 6 und rührt 16 Stunden mit 3,34 g Gadeliniumacetat bei 50 ° C. Die so erhaltene Lösung unterwirft man einer Ionenaustauscherreinigung und erhält 3,62 g der Titelverbindung als farbloses Pulver.

| Analyse: $C_{17}$ $H_{29}$ Gd $N_4$ $O_8$ (574.69) | | | |
|---|---|---|---|
| C 35.53<br>35.68 | H 5.09<br>5.19 | Gd 27.36<br>27.03 | N 9.75 (ber.)<br>9.68 (gef.) |

Beispiel 4

Gadolinium-III-Komplex der N-(2-Aminoethyl)-1,4,7,10-tetraazacyclododecan-N',N'',N'''-triessigsäure

Zu einer Lösung von 4,30 g N,N',N''-Tris-(ethoxycarbonylmethyl) 1,4,7,10-tetraazacyclododecan in 100 ml Dichlormethan gibt man 2,10 g Triethylamin und 1,22 g N-(2-Chlorethyl)-acetamid. Man rührt 18 Stunden bei Raumtemperatur, schüttelt mit Wasser und dampft im Vakuum ein. Den Rückstand rührt man 8 Stunden mit 50 ml 1 N Natronlauge bei 60 ° C, stellt mit 2 N Salzsäure auf pH 6 und rührt 16 Stunden mit 3,34 g Gadoliniumacetat bei 50 ° C. Die so erhaltene Lösung unterwirft man einer Ionenaustauscherreinigung und erhält 3,21 g der Titelverbindung als farbloses Pulver.

| Analyse: $C_{16}$ $H_{28}$ Gd $N_5$ $O_6$ (543.68) | | | |
|---|---|---|---|
| C 35.35<br>35.17 | H 5.19<br>5.45 | Gd 28.92<br>28.68 | N 12.88 (ber.)<br>12.81 (gef.) |

Beispiel 5

Bis-Gadolinium-III-Komplex der 1,1'-(1,3-Propylen)-bis(1,4,7,10-tetraazacyclododecan-4,7,10-triessigsäure)

Eine Lösung von 8,60 g N,N',N''-Tris-(ethoxycarbonylmethyl)1,4,7,10-tetraazacycledodecan und 4.2 g Triethylamin in 200 ml Dichlormethan versetzt man mit 2,02 g 1,3-Dibrompropan und rührt 20 Stunden bei Raumtemperatur, schüttelt mit Wasser und Sole und dampft im Vakuum ein. Den Rückstand filtriert man mit Dichlormethan über 150 g Kieselgel und dampft ein. Man erhält ein zähes Ol, das man mit 60 ml 1 N Natronlauge 16 Stunden rührt, dann mit 100 ml Wasser verdünnt und mit 2 N Salzsäure auf pH 6 einstellt. Nach Zugabe von 6,68 g Gadoliniumacetat rührt man 16 Stunden bei 50 ° C und reinigt die Lösung über Anionen- und Kationenaustauscher. Man erhält 6,56 g der Titelverbindung als farbloses Pulver.

| Analyse: $C_{31}$ $H_{50}$ $Gd_2$ $N_8$ $O_{12}$ (1041.28) | | | |
|---|---|---|---|
| C 35.76<br>35.71 | H 4.84<br>4.58 | Gd 30.20<br>29.94 | N 10.76 (ber.)<br>10.88 (gef.) |

Beispiel 6

Bis-Gadolinium-III-Komplex der Succinyl-bis(1,4,7,10-tetraazacyclododecan-4,7,10-triessigsäure)

Eine Lösung von 4,30 g N,N',N''-Tris-(ethoxycarbonylmethyl)1,4,7,10-tetraazacyclododecan und 4,20 g Triethylamin in 100 ml Dichlormethan versetzt man bei 0°C tropfenweise mit 1,705 g Bernsteinsäuredichlorid, gelöst in 20 ml Dichlormethan, und rührt anschließend 1 Stunde bei Raumtemperatur. Man schüttelt mit Natriumhydrogencarbonatlösung und Sole und dampft im Vakuum ein. Den Rückstand chromatographiert man an 100 g Kieselgel mit Dichlormethan/Ethylacetat (0 - 30 %).

Man erhält ein zähes Ol, das mit 40 ml 1 N Natronlauge 4 Stunden gerührt wird, dann mit Wasser (100 ml) verdünnt und mit 2 N Salzsäure auf pH 6 eingestellt wird. Nach Zugabe von 3,34 g Gadoliniumacetat rührt man 16 Stunden bei 50°C und reinigt die Lösung über Ionenaustauscher. Man erhält 3,90 g der Titelverbindung als weißes Pulver.

| Analyse: $C_{32}H_{48}Gd_2N_8O_{14}$ (1083.28) | | | |
|---|---|---|---|
| C 35.48<br>35.31 | H 4.47<br>4.18 | Gd 29.03<br>28.81 | N 10.34 (ber.)<br>10.57 (gef.) |

Beispiel 7

N-(2-Hydroxyethyl)-1,4,7,10-tetraazacyclododecan-N',N'',N''' triessigsäure

Eine Lösung von 6,46 g N,N',N''-Tris-(ethoxycarbonylmethyl)1,4,7,10-tetraazacyclododecan in 150 ml Dichlormethan versetzt man mit 3,2 g Triethylamin und 1,45 g 2-Chlorethanol. Man rührt 4 Stunden bei Raumtemperatur, schüttelt dann mit Natriumhydrogencarbonatlösung und Sole, trocknet über Magnesiumsulfat und dampft im Vakuum ein. Den Rückstand rührt man 16 Stunden mit 60 ml 1 N Natronlauge. Durch Zugabe von 5 N Salzsäure wird auf pH 2,5 eingestellt. Die erhaltene Suspension gibt man auf einen Ionenaustauscher (DOWEX 50W-X4 in der $H^+$-Form), eluiert mit Wasser und anschließend mit 0,5 M $NH_3$-Lösung. Man engt im Vakuum ein und isoliert die Titelverbindung durch Zugabe von Ethanol und Absaugen des Niederschlags. Man erhält 4,24 g der Titelverbindung, die Reinheit wird durch pH-Titration und Elementaranalyse überprüft.

| Analyse: $C_{16}H_{30}N_4O_7$ (390.44) | | |
|---|---|---|
| C 49.22<br>49.48 | H 7.75<br>7.83 | N 14.35 (ber.)<br>14.09 (gef.) |

Beispiel 8

N-[N-(2-Hydroxyethyl)-carbamoylmethyl]-1,4,7,10-tetraazacyclododecan-N',N'',N'''-triessigsäure

Eine Lösung von 12,50 g N,N',N''-Tris-(ethoxycarbonylmethyl)1,4,7,10-tetraazacyclododecan-N'''-essigsäure in 250 ml Dichlormethan versetzt man bei 0 °C mit 5,22 g Triethylamin und anschließend mit 3,50 g Chlorameisensäureisobutylester. Nach 1 Stunde tropft man 1,60 g Ethanolamin, gelöst in 50 ml Dichlormethan, hinzu, rührt 2 Stunden bei Raumtemperatur, schüttelt mit Sodalösung und Sole, trocknet über Magnesiumsulfat und dampft im Vakuum ein. Den Rückstand chromatographiert man mit Chloroform/Aceton (10:1) an 200 g Kieselgel und erhält 11 g N-(2-Hydroxyethyl)carbamoylmethyl-N',N'',N'''-(tris-ethoxycarbonylmethyl)-1,4,7,10 tetraazacyclododecan.

Dieses Produkt rührt man mit 100 ml 1 N Natronlauge 5 Stunden bei Raumtemperatur, säuert dann mit verdünnter Salzsäure auf pH 2,5 an und reinigt die Suspension an einem Kationenaustauscher (DOWEX 50W-X4), wobei man mit Wasser und anschließend mit 0,5 M $NH_3$-Lösung eluiert. Das Eluat wird weitgehend eingeengt, und nach Zugabe von Ethanol kristallisiert die Titelverbindung aus, die man durch Filtration isoliert. Man erhalt 7,2 g der Titelverbindung, deren Reinheit durch Titratien und Elementaranalyse untersucht wird.

| Analyse: $C_{18}$ $H_{33}$ $N_5$ $O_8$ (447.49) | | |
|---|---|---|
| C 48.31<br>48.20 | H 7.43<br>7.48 | N 15.65 (ber.)<br>15.49 (gef.) |

Beispiel 9

Gadoliniumkomplex der N-(Morpholinocarbenylmethyl)-1,4,7,10-tetraazacyclododecan-N',N'',N'''-triessigsäure

55,77 g N-(Morpholinocarbonylmethyl)-N',N'',N'''-tris-(ethoxycarbonylmethyl)-1,4,7,10-tetraazacycledodecan werden analog Beispiel 1 mit Natronlauge hydrolysiert und mit Gadoliniumacetat komplexiert. Man erhält 45,19 g (72 % d. Theorie) der Titelverbindung als weißes Pulver.

Das Ausgangsmaterial für die Herstellung der Titelverbindung erhält man, indem man analog nach Beispiel 2b verfährt, aber Morpholin an Stelle von N-Methylamino-2,3-propandiol verwendet.

| Analyse: $C_{20}$ $H_{32}$ Gd $N_5$ $O_8$ (627.75) | | | |
|---|---|---|---|
| C 38.27<br>38.02 | H 5.14<br>5.09 | Gd 25.04<br>24.83 | N 11.16 (ber.)<br>11.35 (gef.) |

Beispiel 10

Gadoliniumkomplex der N-(Morpholinocarbonylmethyl)-1,4,7,10-tetraazacyclododecan-N',N'',N'''-tris-(2-methyl-essigsäure)

53.06 g N-(Morpholinocarbonylmethyl)-N',N'',N'''-tris-(1-ethoxycarbonyl-1-ethyl)-1,4,7,10-tetraazacyclododecan werden analog Beispiel 2 mit Natronlauge hydrolysiert und mit Gadoliniumacetat komplexiert. Man erhält 52,20 g (78 % d. Theorie) der Titelverbindung als weißes Pulver.

| Analyse: $C_{23}$ $H_{38}$ Gd $N_5$ $O_8$ (669.83) | | | |
|---|---|---|---|
| C 41.24<br>41.35 | H 5.72<br>5.65 | Gd 23.48<br>23.33 | N 10.46 (ber.)<br>10.62 (gef.) |

Beispiel 11

Bis-Gadoliniumkomplex der 1,1'-(2-Hydroxy-1,3-propylen)-bis-(1,4,7,10-tetraazacyclododecan-4,7,10-triessigsäure

Zu einer Lösung von 43,05 g N,N',N''-Tris-(ethoxy-carbonylmethyl)-1,4,7,10 tetraazacyclododecan in 450 ml Dimethylformid gibt man 4,63 g Epichlorhydrin. Nach 1 Stunde versetzt man mit 7,5 g Natriumjodid und erhitzt 24 Stunden auf 80 ° C. Man engt im Vakuum ein, verteilt den Rückstand zwischen Wasser und Chloroform, trocknet die Chloroformphase über Natriumsulfat und dampft im Vakuum ein. Den Rückstand chromatographiert man an 1 kg Kieselgel mit Dichlormethan - 10 % Aceton und erhält 27,5 g 1,1'-(2-Hydroxy-1,3-propylen)-bis-(1,4,7,10-tetraazacyclododecan-triessigsäureethylester) als zähes Öl.

Man löst 9,17 g des so hergestellten Esters in 200 ml Ethanol und rührt mit 30 ml 3 N Natronlauge 20 Stunden bei Raumtemperatur, stellt dann mit Salzsäure auf pH 6 und rührt 16 Stunden bei 60 ° C mit 6,68 g Gadoliniumacetat und reinigt die Lösung über Anionen- und Kationenaustauscher und erhält 10,05 g der Titelverbindung als weißes Pulver.

| Analyse: $C_{31}$ $H_{50}$ $Gd_2$ $N_8$ $O_{13}$ (1057.28) | | | |
|---|---|---|---|
| C 35.22<br>35.03 | H 4.77<br>4.89 | Gd 29.75<br>29.49 | N 10.60 (ber.)<br>10.41 (gef.) |

Beispiel 12

N-Methylglucaminsalz des Mangan-II-Komplexes der N-[N-(2-Hydroxyethyl)-carbamoylmethyl]-1,4,7,10-tetraazacyclododecan-N',N'',N'''-triessigsäure

8,95 g (20 mMol)-N-[N-(2-Hydroxyethyl)-carbamoylmethyl]-1,4,7,10-tetraazacyclododecan-N',N'',N'''-triessigsäure werden in 30 ml Wasser suspendiert und mit 1,40 g (20 mMol) Mangan-II-oxid 3 Stunden auf 100°C erhitzt. Man versetzt dann mit 3,90 g (20 mMol) N-Methylglucamin, erhitzt weitere 12 Stunden auf 100°C und dampft die Lösung im Vakuum zur Trockne. Man erhält 13,8 g der Titelverbindung als rosafarbenes Pulver,
Fp. 140-143°C.

| Analyse: $C_{25}$ $H_{48}$ Mn $N_6$ $O_{13}$ (695.64) | | | |
|---|---|---|---|
| C 43.17<br>43.44 | H 6.96<br>7.16 | Mn 7.90<br>7.69 | N 12.08 (ber.)<br>12.01 (gef.) |

Beispiel 13

Dysprosium-III-Komplex der N-(Morpholinocarbonylmethyl)-1,4,7,10-tetraazacyclododecan-N',N'',N'''-triessigsäure

20 g N-(Morpholinocarbonylmethyl)-N',N'',N'''-tris-(ethoxycarbonylmethyl)-1,4, 7,10-tetraazacyclododecan werden analog Beispiel 2 mit Natronlauge hydrolysiert und mit Dysprosiumacetat komplexiert. Man erhält 16,3 g der Titelverbindung als weißes Pulver.

| Analyse: $C_{20}$ $H_{32}$ Dy $N_5$ $O_8$ (633.01) | | | |
|---|---|---|---|
| C 37.95<br>37.71 | H 5.10<br>4.92 | Dy 25.67<br>25.81 | N 11.06 (ber.)<br>11.32 (gef.) |

Beispiel 14

Herstellung von mit Gadolinium-N-(Morpholinocarbonylmethyl)-1,4,7,10-tetraazacyclododecan-N',N'',N'''-triessigsäure beladenen Liposomen

Nach der in Proc. Natl. Acad. Sci. U.S.A. 75, 4194 beschriebenen Vorgehensweise wird ein Lipidgemisch aus 75 Mol % Ei-Phosphatidylcholin und 25 Mol % Cholesterol als Trockensubstanz hergestellt. Hiervon werden 500 mg in 30 ml Diethylether gelöst und im Ultraschall-Bad tropfenweise mit 3 ml einer wässrigen 0,1 M Lösung des Gadoliniumkomplexes der N-(Morpholinocarbonylmethyl)-1,4,7,10-tetraaza-N',N'',N'''-triessigsäure versetzt. Man setzt die Ultrabeschallung noch 10 Minuten fort und engt im Vakuum ein. Der gelartige Rückstand wird in 0,125 M Natriumchloridlösung suspendiert und bei 0°C wiederholt bei 20 000 g zentrifugiert zur Abtrennung von nichtverkapseltem Gadoliniumkomplex. Man unterwirft die Suspension dann einer Gefriertrocknung in Multivials. Die Applikation erfolgt als kolloidale Dispersion in 0.9 %iger Natriumchloridlösung.

Beispiel 15

Herstellung einer Lösung des Yttrium-90-Komplexes vom Konjugat der 1,4,7,10-Tetraazacyclododecan-N,N',N'',N'''-tetraessigsäure mit monoklonalen Antikörper

Zu einer Suspension von 4 mg 1,4,7,10-Tetraazacyclododecan-N,N',N'',N'''-Tetraazacyclododecan-N,N',N'',N'''-tetraessigsäure in 1 ml Wasser gibt man 2 mg N-(3-Dimethylaminopropyl)-N'-ethyl-carbodiimid-hydrochlorid und anschließend 1 ml einer Lösung von 0,6 mg monoklonalem Antikörper (mit Spezifität gegen Melanoma-Antigen) in 0,05 molarem Natriumhydrogencarbonatpuffer (pH 7,8) gelöst. Man rührt 2 Stunden bei Raumtemperatur und dialysiert gegen einen 0,3 M Natriumphosphatpuffer. Dann setzt man 1 ml einer Yttrium-90 Lösung in Acetatpuffer pH 6 zu (hergestellt nach Int. J. Appl. Radiat. Isot., Vol 36 [1985], S. 803) und inkubiert 24 Stunden bei Raumtemperatur. Die Lösung wird über eine Sephadex G 25-Säule gegeben und die radioaktive Proteinfraktion steril filtriert und in Multivials abgefüllt. Durch Lyophilisierung wird ein lagerfähiges Trockenpräparat erhalten.

Beispiel 16

Indium-111-Komplex der N-(morpholinocarbonylmethyl)-1,4,7,10-tetraazacyclododecan-N',N'',N'''-triessigsäure

Man verfahrt analog Beispiel 9 und komplexiert mit radioaktivem $^{111}$-Indiumchlorid. Zur Uberprüfung, ob die Metallionen vollständig als Chelat gebunden sind, untersucht man die Lösung der Titelverbindung dünnschichtchromatographisch an Kieselgelplatten in dem System Methanol-Wasser (2:1) Nicht-chelatisierte Metallionen werden dabei als radioaktive Zone am Startfleck erkannt. Falls notwendig wird die Chelatisierung durch weitere Zugabe von N-(Morpholinocarbonylmethyl)-N',N'',N'''-tris-(ethoxycarbonylmethyl)-1,4,7,10-tetraazacyclododecan und anschließende Esterspaltung vervollständigt.

Auf die gleiche Weise erhält man den Gadolinium-153-Komplex der N-(Morpholinocarbonylmethyl)-1,4,7,10-tetraazacyclododecan-N',N'',N'''-triessigsäure.

**Patentansprüche**

**1.** 1,4,7,10-Tetraazacyclododecan-Derivate der allgemeinen Formel I

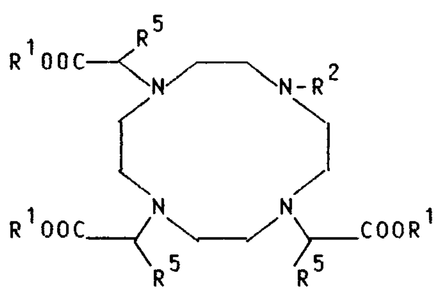

(I),

worin

- $R^1$ unabhängig voneinander Wasserstoff oder ein Metallionenäquivalent,
- $R^5$ Wasserstoff, eine Methyl- oder Ethylgruppe,
- $R^2$ eine gerade oder verzweigte, gesättigte oder ungesättigte, durch 1 bis 5 Hydroxy- oder $C_1$-$C_4$-Alkoxygruppen substituierte Alkylgruppe mit bis 16 Kohlenstoffatomen,
  $-CH_2$-X-V mit X in der Bedeutung von Carbonyl,
  einer geradkettigen oder verzweigtkettigen Alkylengruppe mit 1 bis 10 Kohlenstoffatomen, die gegebenenfalls durch 1 bis 5 Hydroxy- oder $C_1$-$C_4$-Alkoxygruppen substituiert ist, oder einer gerad- oder verzweigtkettigen durch Sauerstoffatome unterbrochenen Alkylengruppe mit 2 bis 23 Kohlenstoffatomen,
- V in der Bedeutung von

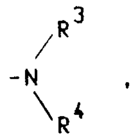

wobei $R^3$ und $R^4$ unabhängig voneinander Wasserstoff, eine gerade oder verzweigte, gegebenenfalls durch 1 bis 5 Hydroxy- oder $C_1$-$C_4$-Alkoxygruppen substituierte Alkylgruppe mit bis 16 Kohlenstoffatomen oder $R^3$ und $R^4$ gemeinsam mit dem Stickstoffatom einen Pyrrolidin-, Piperidin-, Morpholin- oder Piperazinring darstellen,

oder

$R^2$ oder $R^3$ einen über eine 2-20 Kohlenstoffatome enthaltende Alkylenkette, die gegebenenfalls an den Enden Carbonylgruppen trägt und gegebenenfalls durch 1 bis 4 Sauerstoffatome unterbrochen oder durch 1 bis 5 Hydroxy-, $C_1$-$C_4$-Alkoxy- oder Carboxy-$C_1$-$C_4$-alkyl-Gruppen substituiert ist, gebundenen zweiten Makrozyklus der Formel I'

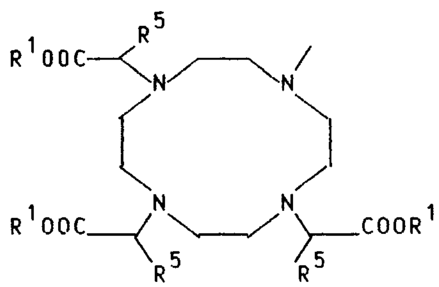

(I'),

oder $R^2$ B oder $CH_2$-COB, wobei B für ein sich in dem zu untersuchenden Organ oder Organteil bzw. im Tumor besonders anreicherndes Makro- oder Biomelekül steht,

und im Molekül vorhandene funktionelle Gruppen gewünschtenfalls als Konjugat an Biomoleküle gekoppelt sind sowie deren Salze mit organischen oder anorganischen Basen oder Aminosäuren bzw. mit anorganischen oder organischen Säuren.

**2.** Verbindungen nach Anspruch 1, dadurch gekennzeichnet, daß $R^1$ jeweils für ein Wasserstoffatom steht.

**3.** Verbindungen nach Anspruch 1, dadurch gekennzeichnet, daß mindestens zwei der Substituenten $R^1$ Metallionenäquivalente mindestens eines Metalls der Ordnungszahlen 21 bis 29, 42, 44 oder 57 bis 70 sind.

**4.** Verbindungen nach Anspruch 1, dadurch gekennzeichnet, daß mindestens zwei der Substituenten $R^1$ Metallionenäquivalente mindestens eines Radionuklids eines Elements der Ordnungszahen 27, 29, 31, 32, 38, 39, 43, 49, 64, 70 oder 77 sind.

**5.** N-[2,3-Dihydroxy-N-methyl-propylcarbamoylmethyl]-1,4,7,10-tetraazacyclododecan-N',N'',N'''-triessigsäure,

N-[N-Ethylcarbamoylmethyl]-1,4,7,10-tetraazacyclododocan-N',N'',N'''-triessigsäure,

N-(2,3-Dihydroxy-1-propyl)-1,4,7,10-tetraazacyclododecan-N',N'',N'''-triessigsäure,

N-(2-Aminoethyl)-1,4,7,10-tetraazacyclododecan-N',N'',N'''-triessigsäure,

N-(2-Hydroxyethyl)-1,4,7,10-tetraazacyclododecan-N',N'',N'''-triessigsäure,

N-[N-(2-Hydroxyethyl)-carbamoylmethyl]-1,4,7,10-tetraazacyclododecan-N',N'',N'''-triessigsäure,

N-(Morpholinocarbonylmethyl)-1,4,7,10-tetraazacyclododecan-N',N'',N'''-triessigsäure,

N-(Morpholinocarbonylmethyl)-1,4,7,10-tetraazacyclododecan-N',N'',N'''-tris-(2-methyl-essigsäure),

und die Komplexe dieser Verbindungen mit paramagnetischen Metallen gemäß Anspruch 2 oder Radioisotopen gemäß Anspruch 4.

**6.** 1,1'-(1,3-Propylen)-bis(1,4,7,10-tetraazacyclododecan 4,7,10-triessigsäure),

Succinyl-bis(1,4,7,10-tetraazacyclododecan-4,7,10-triessigsäure),

1,1'-(2-Hydroxy-1,3-propylen)-bis-(1,4,7,10-tetraazacyclododecan-4,7,10-triessigsäure,

und die Komplexe dieser Verbindungen mit paramagnetischen Metallen gemäß Anspruch 3 oder Radioisotopen gemäß Anspruch 4.

**7.** Gadolinium-, Mangan- und Dysprosium-Komplexe von den in den Ansprüchen 5 und 6 genannten Komplexbildnern.

**8.** Pharmazeutische Mittel, enthaltend mindestens einen Metallkomplex der allgemeinen Formel I mit mindestens zwei der Substituenten $R^1$ in der Bedeutung eines Metallionenäquivalents, gegebenenfalls in Form von Liposomen und gegebenenfalls mit den in der Galenik üblichen Zusätzen.

**9.** Verwendung der Verbindungen nach Anspruch 2 als Komplexbildner.

**10.** Verwendung von mindestens einem Metallkomplex der allgemeinen Formel I mit mindestens zwei der Substituenten $R^1$ in der Bedeutung eines Metallionenäquivalents für die Herstellung von Mitteln für die NMR-, Röntgen-, Ultraschall- oder Radiodiagnostik und Radio-Therapie.

**11.** Verfahren zur Herstellung von 1,4,7,10-Tetraazacyclododecan-Derivaten nach Anspruch 1 bis 3, dadurch gekennzeichnet, daß man in an sich bekannter Weise in Verbindungen der allgemeinen Formel II

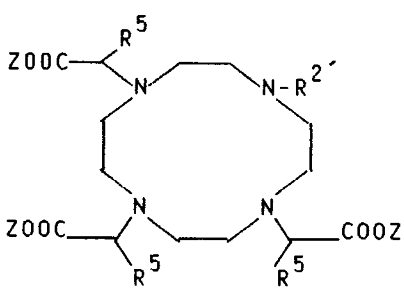

(II),

worin

$R^5$ die oben angegebene Bedeutung hat,

$R^{2'}$ die für $R^2$ angegebene Bedeutung hat, jedoch nicht für ein Makro- oder Biomolekül B oder die Gruppe $CH_2$-COB stehen soll und

Z Carboxylschutzgruppen bedeuten,

die Schutzgruppen Z abspaltet und die so erhaltenen Säuren ($R^1$ der allgemeinen Formel I steht für Wasserstoff) gewünschtenfalls

a) in an sich bekannter Weise mit mindestens einem Metalloxid oder Metallsalz eines Elements der Ordnungszahlen 21-29, 31, 32, 38, 39, 42-44, 49, 57-70 oder 77 umsetzt und anschließend, falls gewünscht, vorhandene acide Wasserstoffatome mit anorganischen und/oder organischen Basen oder Aminosäuren und vorhandene basische Gruppen mit anorganischen oder organischen Säuren in physiologisch verträgliche Salze überführt,

oder

b) in an sich bekannter Weise mit mindestens einem Metalloxid oder Metallsalz eines Elements der Ordnungszahlen 21-29, 31, 32, 38, 39, 42-44, 49, 57 bis 70 oder 77 umsetzt und anschließend die so

erhaltenen Metallkomplexe in an sich bekannter Weise über im Molekül enthaltene funktionelle Gruppen oder an $R^2$ bzw. an die in $R^2$ enthaltene CO-Gruppe ein Makromolekül bindet und, falls gewünscht, vorhandene acide Wasserstoffatome mit anorganischen und/oder organischen Basen oder Aminosäuren und vorhandene basische Gruppen mit anorganischen oder organischen Säuren in physiologisch verträgliche Salze überführt,
oder
c) in an sich bekannter Weise über die im Molekül enthaltenen funktionellen Gruppen oder an $R^2$ bzw. an die in $R^2$ enthaltene CO-Gruppe ein Makromolekül bindet und anschließend in an sich bekannter Weise mit mindestens einem Metalloxid oder Metallsalz eines Elements der Ordnungszahlen 21-29, 31, 32, 38, 39, 42-44, 49, 57-70 oder 77 umsetzt und anschließend, falls gewünscht, vorhandene acide Wasserstoffatome mit anorganischen und/oder organischen Basen oder Aminosäuren und vorhandene basische Gruppen mit anorganischen oder organischen Säuren in physiologisch verträgliche Salze überführt.

**12.** Verfahren zur Herstellung der pharmazeutischen Mittel gemäß Anspruch 8, dadurch gekennzeichnet, daß man die in Wasser oder physiologischer Salzlösung gelöste oder suspendierte Komplexverbindung, gegebenenfalls mit den in der Galenik üblichen Zusätzen in eine für die enterale oder parenterale Applikation geeignete Form bringt.

## Claims

**1.** 1,4,7,10-tetraazacyclododecane derivatives of the general formula I

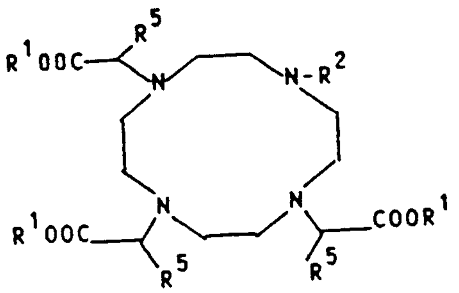

(I)

wherein

each $R^1$, independently of the others, represents hydrogen or a metal ion equivalent,

$R^5$ represents hydrogen or a methyl or ethyl group,

$R^2$ represents a linear or branched, saturated or unsaturated alkyl group that has up to 16 carbon atoms and that is substituted by from 1 to 5 hydroxy or $C_1$-$C_4$-alkoxy groups, $-CH_2$-X-V in which X represents carbonyl, a linear or branched alkylene group having from 1 to 10 carbon atoms that is optionally substituted by from 1 to 5 hydroxy or $C_1$-$C_4$-alkoxy groups, or a linear or branched alkylene group that is interrupted by oxygen atoms and has from 2 to 23 carbon atoms,
V represents

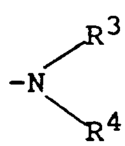

,

in which each of $R^3$ and $R^4$, independently of the other, represents hydrogen, a linear or branched alkyl group that has up to 16 carbon atoms and that is optionally substituted by from 1 to 5 hydroxy or $C_1$-$C_4$-alkoxy groups, or $R^3$ and $R^4$ together with the nitrogen atom represent a pyrrolidine, piperidine, morpholine or piperazine ring,

or

$R^2$ or $R^3$ represents a second macrocycle of the formula I'

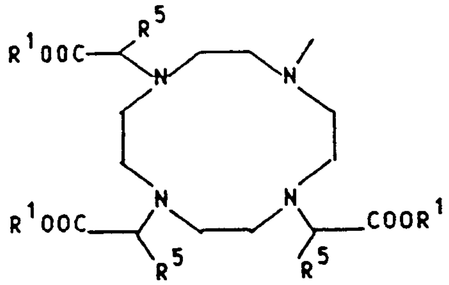

(I')

that is linked via an alkylene chain that has from 2 to 20 carbon atoms and optionally carries carbonyl groups at its ends and is optionally interrupted by from 1 to 4 oxygen atoms or substituted by from 1 to 5 hydroxy, $C_1$-$C_4$-alkoxy or carboxy-$C_1$-$C_4$-alkyl groups,
or $R^2$ represents B or $CH_2$-COB, B representing a macromolecule or biomolecule that accumulates to an especially great extent in the organ or organ part to be investigated or in the tumour,

and functional groups present in the molecule are, if desired, conjugated with biomolecules, and the salts thereof with organic or inorganic bases or amino acids or with inorganic or organic acids.

2. Compounds according to claim 1, characterised in that each $R^1$ represents a hydrogen atom.

3. Compounds according to claim 1, characterised in that at least two of the substituents $R^1$ are metal ion equivalents of at least one metal of atomic number 21 to 29, 42, 44 or 57 to 70.

4. Compounds according to claim 1, characterised in that at least two of the substituents $R^1$ are metal ion equivalents of at least one radionuclide of an element of atomic number 27, 29, 31, 32, 38, 39, 43, 49, 64, 70 or 77.

5. N-[2,3-dihydroxy-N-methylpropylcarbamoylmethyl]-1,4,7,10-tetraazacyclododecane-N',N'',N'''-triacetic acid,

N-[N-ethylcarbamoylmethyl]-1,4,7,10-tetraazacyclododecane-N',N'',N'''-triacetic acid,

N-(2,3-dihydroxy-1-propyl)-1,4,7,10-tetraazacyclododecane-N',N'',N'''-triacetic acid,

N-(2-aminoethyl)-1,4,7,10-tetraazacyclododecane-N',N'',N'''-triacetic acid,

N-(2-hydroxyethyl)-1,4,7,10-tetraazacyclododecaneN',N'',N'''-triacetic acid,

N-[N-(2-hydroxyethyl)-carbamoylmethyl]-1,4,7,10-tetraazacyclododecane-N',N'',N'''-triacetic acid,

N-(morpholinocarbonylmethyl)-1,4,7,10-tetraazacyclododecane-N',N'',N'''-triacetic acid,

N-(morpholinocarbonylmethyl)-1,4,7,10-tetraazacyclododecane-N',N'',N'''-tris-(2-methylacetic acid),

and the complexes of those compounds with paramagnetic metals according to claim 2 or radioisotopes according to claim 4.

6. 1,1'-(1,3-propylene)-bis(1,4,7,10-tetraazacyclododecane-4,7,10-triacetic acid),

succinyl-bis(1,4,7,10-tetraazacyclododecane-4,7,10-triacetic acid),

1,1'-(2-hydroxy-1,3-propylene)-bis(1,4,7,10-tetraazacyclododecane-4,7,10-triacetic acid),

and the complexes of those compounds with paramagnetic metals according to claim 3 or radioisotopes according to claim 4.

**7.** Gadolinium, manganese and dysprosium complexes of the complexing agents mentioned in claims 5 and 6.

**8.** Pharmaceutical agents containing at least one metal complex of the general formula I in which at least two of the substituents $R^1$ represent a metal ion equivalent, optionally in the form of liposomes and optionally with the adjuvants customarily used in galenical pharmacy.

**9.** Use of the compounds according to claim 2 as complexing agents.

**10.** Use of at least one metal complex of the general formula I in which at least two of the substituents $R^1$ represent a metal ion equivalent for the preparation of media for NMR diagnostics, X-ray diagnostics, ultrasound diagnostics or radiodiagnostics and radiotherapy.

**11.** Process for the preparation of 1,4,7,10-tetraazacyclododecane derivatives according to claims 1 to 3, characterised in that, in a manner known per se, in compounds of the general formula II

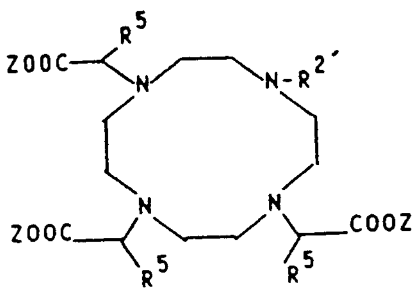

(II)

wherein

$R^5$ is as defined above,

$R^{2'}$ has the meaning given for $R^2$, but is not to represent a macromolecule or biomolecule B or the group $CH_2$-COB, and

Z represents carboxy-protecting groups,

the protecting groups Z are split off and the resulting acids ($R^1$ in the general formula I represents hydrogen) are, if desired,

a) reacted in a manner known per se with at least one metal oxide or metal salt of an element of atomic number 21 to 29, 31, 32, 38, 39, 42 to 44, 49, 57 to 70 or 77 and then, if desired, acidic hydrogen atoms present are converted with inorganic and/or organic bases or amino acids and basic groups present are converted with inorganic or organic acids into physiologically tolerable salts,

or

b) reacted in a manner known per se with at least one metal oxide or metal salt of an element of atomic number 21 to 29, 31, 32, 38, 39, 42 to 44, 49, 57 to 70 or 77 and then the resulting metal complexes are linked in a manner known per se via functional groups contained in the molecule or to $R^2$ or to the CO group contained in $R^2$ a macromolecule and, if desired, acidic hydrogen atoms present are converted with inorganic and/or organic bases or amino acids and basic groups present are converted with inorganic or organic acids into physiologically tolerable salts,

or

c) linked in a manner known per se via the functional groups contained in the molecule or to $R^2$ or to the CO group contained in $R^2$ a macromolecule and then reacted, in a manner known per se, with at least one metal oxide or metal salt of an element of atomic number 21 to 29, 31, 32, 38, 39, 42 to 44, 49, 57 to 70 or 77 and then, if desired, acidic hydrogen atoms present are converted with inorganic and/or organic bases or amino acids and basic groups present are converted with inorganic or organic acids into physiologically tolerable salts.

**12.** Process for the preparation of the pharmaceutical agents according to claim 8, characterised in that the complex compound dissolved or suspended in water or physiological saline solution is made into a form suitable for enteral or parenteral administration, optionally together with adjuvants customarily used in galenical pharmacy.

## Revendications

**1.** Dérivés de 1,4,7,10-tétraazacyclododécane de la formule générale I

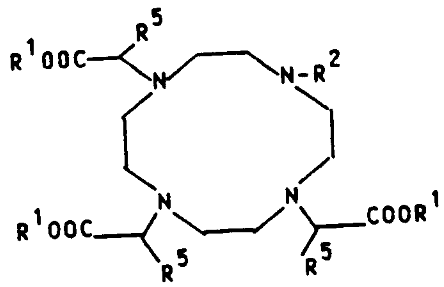

(I),

dans laquelle

$R^1$ représente, indépendamment, de l'hydrogène ou un équivalent d'ion métallique,

$R^5$ représente de l'hydrogène ou un groupe méthyle ou éthyle,

$R^2$ représente un groupe alkyle comportant jusqu'à 16 atomes de carbone, linéaire ou ramifié, saturé ou insaturé, substitué par 1 à 5 groupes hydroxy ou alcoxy en $C_1$-$C_4$, un groupement $-CH_2$-X-V où X représente du carbonyle, un groupe alkylène linéaire ou ramifié comportant 1 à 10 atomes de carbone, qui est éventuellement substitué par 1 à 5 groupes hydroxy ou alcoxy en $C_1$-$C_4$, ou un groupe alkylène linéaire ou ramifié, interrompu par des atomes d'oxygène et comportant 2 à 23 atomes de carbone,

V représentant un groupement

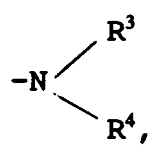

où $R^3$ et $R^4$ représentent indépendamment l'un de l'autre de l'hydrogène, un groupe alkyle comportant jusqu'à 16 atomes de carbone, linéaire ou ramifié, éventuellement substitué par 1 à 5 groupes hydroxy ou alcoxy en $C_1$-$C_4$, $R^3$ et $R^4$ pouvant former conjointement avec l'atome d'azote un noyau de pyrrolidine, de pipéridine, de morpholine ou de pipérazine,

$R^2$ ou $R^3$ pouvant représenter un deuxième macrocycle de la formule I'

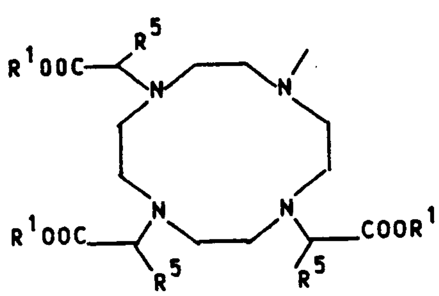

(I'),

qui est lié par l'intermédiaire d'une chaîne alkylène comportant de 2 à 20 atomes de carbone qui porte éventuellement aux extrémités des groupes carbonyle et est éventuellement interrompue par 1 à 4 atomes d'oxygène ou est substituée par 1 à 5 groupes hydroxy, alcoxy en $C_1$-$C_4$ ou carboxy-$C_1$-$C_4$-alkyle,

$R^2$ pouvant représenter B ou un groupement $CH_2$-COB, B représentant une macromolécule ou biomolécule qui s'enrichit particulièrement dans l'organe ou la partie d'organe ou respectivement dans la tumeur à examiner,

des groupes fonctionnels présents dans la molécule pouvant éventuellement être couplés sur des biomolécules sous la forme d'un produit de conjugaison,

ainsi que leurs sels avec des bases organiques ou inorganiques ou des amino-acides et respectivement avec des acides inorganiques ou organiques.

2. Composés suivant la revendication 1, caractérisés en ce que $R^1$ représente respectivement un atome d'hydrogène.

3. Composés suivant la revendication 1, caractérisés en ce qu'au moins deux des substituants $R^1$ sont des équivalents d'ions métalliques d'au moins un métal des nombres atomiques 21 à 29, 42, 44 ou 57 à 70.

4. Composés suivant la revendication 1, caractérisés en ce qu'au moins deux des substituants $R^1$ représentent des équivalents d'ions métalliques d'au moins un radionuclide d'un élément des nombres atomiques 27, 29, 31, 32, 38, 39, 43, 49, 64, 70 ou 77.

5. Acide N-[2,3-dihydroxy-N-méthylpropylcarbamoylméthyl]-1,4,7,10-tétraazacyclododécane-N',N'',N'''-triacétique,
acide N-[N-éthylcarbamoylméthyl]-1,4,7,10-tétraazacyclododécane-N',N'',N'''-triacétique,
acide N-(2,3-dihydroxy-1-propyl)-1,4,7,10-tétraazacyclododécane-N',N'',N'''-triacétique,
acide N-(2-aminoéthyl)-1,4,7,10-tétraazacyclododécane-N',N'',N'''-triacétique,
acide N-(2-hydroxyéthyl)-1,4,7,10-tétraazacyclododécane-N',N'',N'''-triacétique,
acide N-[N-(2-hydroxyéthyl)-carbamoylméthyl]-1,4,7,10-tétraazacyclododécane-N',N'',N'''-triacétique,
acide N-(morpholinocarbonylméthyl)-1,4,7,10-tétraazacyclododécane-N',N'',N'''-triacétique,
acide N-(morpholinocarbonylméthyl)-1,4,7,10-tétraazacyclododécane-N',N'',N'''-tris-(2-méthyl-acétique),
et les complexes de ces composés avec des métaux paramagnétiques suivant la revendication 3 ou des radioisotopes selon la revendication 4.

6. Acide 1,1'-(1,3-propylène)-bis(1,4,7, 10-tétraazacyclododécane-4,7,10-triacétique),
acide succinyl-bis(1,4,7,10-tétraazacyclododécane-4,7,10-triacétique),
acide 1,1'-(2-hydroxy-1,3-propylène)-bis-(1,4,7,10-tétraazacyclododécane-4,7,10-triacétique),
et les complexes de ces composés avec des métaux paramagnétiques selon la revendication 3 ou des radioisotopes selon la revendication 4.

7. Complexes de gadolinium, de manganèse et de dysprosium et des complexants cités dans les revendications 5 et 6.

8. Produits pharmaceutiques, contenant au moins un complexe métallique de la formule générale I avec au moins deux des substituants $R^1$ signifiant un équivalent d'ion métallique, éventuellement sous la forme de liposomes et éventuellement avec les additifs courants en pharmacie galénique.

9. Utilisation des composés suivant la revendication 2, comme complexants.

10. Utilisation d'au moins un complexe métallique de la formule générale I où au moins deux des substituants $R^1$ représentent un équivalent d'ion métallique, pour la préparation de produits de diagnostic par RMN, des rayons X, des ultrasons ou de produits de radiodiagnostic et de radiothérapie.

11. Procédé de préparation de dérivés de 1,4,7,10-tétraazacyclododécane selon l'une des revendications 1 à 3, caractérisé en ce que, d'une manière connue en soi, dans des composés de la formule générale II

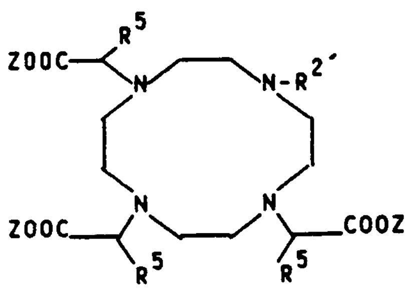

(II),

dans laquelle

$R^5$ a la même signification que celle donnée précédemment,

$R^{2'}$ a la signification donnée pour $R^2$, mais ne peut pas représenter une macromolécule ou biomolécule B ou le groupement $CH_2$-COB, et

Z représente des groupements de protection de carboxyle,

on sépare les groupes de protection Z, et en ce que les acides ainsi obtenus ($R^1$ de la formule générale I représente de l'hydrogène), éventuellement

a) on les fait réagir d'une manière connue en soi avec au moins un oxyde métallique ou sel métallique d'un élément des nombres atomiques 21-29, 31, 32, 38, 39, 42-44, 49, 57-70 ou 77 et ensuite, dans le cas où cela est souhaité, on convertit en sels physiologiquement compatibles des atomes d'hydrogène acides présents par des bases inorganiques et/ou organiques ou des amino-acides et des groupes basiques présents par des acides inorganiques ou organiques, ou

b) d'une manière connue en soi on les fait réagir avec au moins un oxyde métallique ou sel métallique d'un élément des nombres atomiques 21-29, 31, 32, 38, 39, 42-44, 49, 57 à 70 ou 77, et ensuite sur les complexes métalliques ainsi obtenus on fixe d'une manière connue en soi une macromolécule par l'intermédiaire de groupes fonctionnels contenus dans la molécule ou sur $R^2$ ou respectivement sur le groupe CO contenu dans $R^2$ et, dans le cas où cela est souhaité, on convertit en sels physiologiquement compatibles des atomes d'hydrogène acides présents par des bases inorganiques et/ou organiques ou des amino-acides et des groupes basiques présents par des acides inorganiques ou organiques, ou

c) on fixe sur eux d'une manière connue en soi une macromolécule par l'intermédiaire des groupes fonctionnels contenus dans la molécule ou sur $R^2$ ou respectivement sur le groupe CO contenu dans $R^2$, et ensuite, d'une manière connue en soi, on fait réagir avec au moins un oxyde métallique ou sel métallique d'un élément des nombres atomiques 21-29, 31, 32, 38, 39, 42-44, 49, 57-70 ou 77 et ensuite, dans le cas où cela est souhaité, on convertit en sels physiologiquement compatibles des atomes d'hydrogène acides présents par des bases inorganiques et/ou organiques ou des amino-acides et des groupes basiques présents par des acides inorganiques ou organiques.

**12.** Procédé de préparation des produits pharmaceutiques suivant la revendication 8, caractérisé en ce qu'on amène le composé complexe en solution ou suspension dans l'eau ou du sérum physiologique, sous une forme appropriée pour l'application entérale ou parentérale, éventuellement avec les additifs courants en pharmacie galénique.